# EUROPEAN PATENT SPECIFICATION

(11) **EP 3 326 694 B1**
(45) Date of publication and mention of the grant of the patent: **21.11.2018**
(21) Application number: 16201196.9
(22) Date of filing: 29.11.2016
(51) Int. Cl.: A61N 5/10

(54) **SYSTEM AND METHOD FOR ION-BASED RADIOTHERAPY PLANNING**
SYSTEM UND VERFAHREN ZUR IONENBASIERTEN STRAHLENTHERAPIEPLANUNG
SYSTÈME ET PROCÉDÉ DE PLANIFICATION DE RADIOTHÉRAPIE IONIQUE

(43) Date of publication of application: 30.05.2018
(73) Proprietor: RaySearch Laboratories AB, 10365 Stockholm (SE)
(72) Inventor: Traneus, Erik, 75239 Uppsala (SE)

(56) References cited:
- EP-A1- 2 992 930
- WO-A1-2009/139701
- WO-A1-2011/162021
- WO-A1-2016/070938
- US-A1- 2013 303 825
- US-A1- 2015 196 779
- US-A1- 2015 352 374

## Description

### Technical Field

The present invention relates to a system and a method for evaluating an ion-based radiotherapy treatment plan and a computer program product for controlling such a method.

### Background

It is important to evaluate the quality of a radiotherapy treatment plan, to ensure that it will be delivered correctly and affect the patient in the desired way. The delivery of a radiotherapy treatment plan to a patient is affected by a number of factors, an important one being the patient's own anatomy. Structural variations in the patient's body will lead to scattering of particles, altering their path and therefore affecting the resulting dose distribution. The density of the structures traversed by the ion will also affect the path length.

In ion based radiotherapy each ion will emit most of its energy towards the end of its path, creating what is known as the Bragg peak. A key issue in treatment planning is to ensure that the Bragg peaks of all beams are placed within the treatment volume, in such a way that all parts of the treatment volume receive the prescribed dose while minimizing dose to the surrounding volume.

The position of the Bragg peak is affected by the kinetic energy Tp of each ion. The values for Tp are selected so that the ions having the lowest energy will stop in an area at the nearest end of the treatment volume and the ions having the highest energy will stop in the area at the farthest end of the treatment volume.

In ion based radiotherapy the ions follow individual paths through the treatment volume from the point of incidence to the point where the ion has lost all its energy and stops. This point is referred to as the "track end". In ion radiotherapy the distribution of track ends is of great interest as the track ends determine the end of range of the treatment field.

Current methods for delivering ion radiotherapy include:
- The active scanning technique where the patient irradiation is delivered as a sequence of quasi-monoenergetic "spots" whose energy, direction and weight are selected so that the treatment volume is covered. The active technique is sometimes referred to as pencil beam scanning (PBS).
- The passive technique, i.e. where the patient is irradiated by broad fields where the direction, incident energy and lateral extension is modulated so that the treatment volume is covered. The passive technique can be realized by several technical solutions. Examples are double scattering (DS), uniform scanning (US) or wobbling.

The present invention is applicable to all current methods for delivering ion radiotherapy.

Throughout this document both the active scanning technique and the passive technique will be discussed. For the sake of simplicity, these will sometimes be referred to as just PBS and DS, respectively, as the most prominent examples of the respective technique.

There are uncertainty factors due to CT calibration, tissue inhomogeneity, organ motion and deformation. Because of such uncertainty factors there is a desire for a plan to be as robust as possible, meaning that it should provide the same dose distribution even if some factor changes. It is important to evaluate the quality of a radiotherapy treatment plan, to ensure that it will be delivered correctly and affect the patient in the desired way. When evaluating a radiotherapy treatment plan, its robustness is a key factor. The robustness reflects how well the plan will work in the case of small changes to the setup. For example, if the patient receiving the treatment moves relative to the assumed position this will affect where the particles will stop and thereby also the treatment.

US 2017/303825 A1 relates to a method of robust treatment planning, in which a treatment plan is evaluated to see how much the resulting dose distribution will differ if certain parameters that cannot be exactly known, are varied. The parameters may relate to, for example, beam range, patient position and type of tissue. A number of sets of possible parameter values are defined, each set of parameter values typically referred to as a scenario. For each scenario, the resulting dose distribution is determined. If the resulting dose distributions for the different scenarios are very different, that means that the plan is not robust, since a possible variation in parameter values will lead to an unacceptable change in the resulting dose distribution.

### Summary of the invention

It is an object of the present invention to improve and facilitate the evaluation of an ion based radiotherapy treatment plan, in particular the robustness of such a plan.

The invention proposes a method for determining the quality of an ion based treatment plan by evaluating, for each of at least a first and a second portion of a treatment volume, a quality value indicating the spread in a path length value indicative of effective path lengths traversed by ions that stop in that portion, and to use this quality value as a measure of the quality of the plan, in particular, but not only, of its robustness. The effective path length traversed by an ion is a function of the energy of the ion and the density of the matter it traverses. The effective path length can be any suitable measure that allows radiological depth to be compared.

This quality value will be indicative of the robustness of the plan, since for a robust plan the spread in effective path lengths between ions that stop in a particular portion will be small. A greater spread indicates that a small change in the setup may affect the result of the treatment significantly.

Hence, the invention is based on the insight that the amount of scattering and energy loss of ions travelling through a volume will be affected by the structural variations within that volume. A high level of heterogeneity in the volume will lead to a high amount of variation of scattering and energy loss, which means that the particles that stop in a particular portion of the volume will have followed very different paths to get there. This will be reflected by the differences in effective path length traversed by the ions, that is, the spread in effective path lengths will be greater if the volume has a high level of structural variations.

Preferably, the quality value is based on a relationship between the spread in the path length value and the path length values represented by for example the mean of all path length values. In this way, the ratio of the spread to the magnitude of the path length value is taken into account.

In a first preferred embodiment the path length value is based on water equivalent path lengths, WEPL, of ions that stop in the portion and the quality value is based on the spread of water equivalent path lengths, sWEPL. Water equivalent path lengths are a known measure of the effective path lengths traversed by ions, that is available from some dose calculation engines and therefore can be obtained with little additional computational effort.

The initial energy of each ion that stops in a particular portion of the volume can also be used as an indicator of the effective path lengths. In a second preferred embodiment, therefore, the path length value is based on the initial energy of each ion that stops in the portion, and the quality value is based on the spread in initial energy. Information about the initial energy of the ions is also available in many dose calculation engines. It is also possible to base the path length value on both the WEPL and the initial energy for each ion.

The quality value may be calculated based only on the spread of the path length values, or as the spread divided by a mean value representing the path length values. The mean value may be the average value of the path length values of all ions stopping in the portion of the treatment volume, a median value, or any other suitable value indicative of the overall level of the path length values in the portion. It should be understood that when this document mentions the relationship between the quality value and the path length value, the path length value should be taken to mean such an average or median value, or some other value representative of the effective path lengths for ions that stop in the portion considered.

The spread in path length values for a particular voxel may be calculated as a standard deviation of the path length values. Alternatively, the spread may be calculated as a difference in path length values between upper and lower percentiles of the path length values. The ratio between the spread in path length values and the path length values themselves may be importance weighted, such as a dose weighted or weighted with track end distribution.

To achieve this, preferably a dose calculation engine that will obtain WEPL information or initial energy information for each voxel as part of the calculation is used, as this will allow the calculations according to the invention to be performed with little extra computational effort. Such a dose calculation engine is the Monte Carlo engine. Since the quality value in this case is determined from a Monte Carlo propagation through the upstream anatomy, the sensed region per target voxel is sampled in an optimally importance weighted manner with respect to where the ion traverses.

The water equivalent path length is defined as the distance that is equivalent to that measured in water, taking into account the densities of the tissues traversed by the ion and the variation of stopping power ratio between water and the traversed media. It is usually calculated as the product of the distance in the considered materials times the density times the water to medium stopping power ratio. Several methods for determining the water equivalent path length are known.

The portion of the volume is typically a voxel, but it may also be defined in another suitable way, for example, as a group of voxels. To provide an indication of the quality of the plan for the whole volume, preferably all voxels in the volume, or in the relevant part of the volume, are considered, either individually or in groups.

The invention uses this fact to assess plan quality by evaluating, for each portion of the volume, typically for each voxel in which primary protons stop, the spread in water equivalent path length for the ions stopping in that voxel. In particularly preferred embodiments a relationship between the sWEPL and the WEPL is evaluated to obtain the quality value.

The sWEPL and the WEPL may be calculated based on data per voxel accumulated during a forward MC dose calculation.

The ratio sWEPL/WEPL is a measure of the upstream density variation sensed by all protons stopping in the voxel concerned. The ratio sWEPL/WEPL may be used directly as a quality value, recognizing that the resulting value will increase if the spread increases. Alternatively, a dose-weighted sWEPL/WEPL ratio may be used, to give more importance to robustness in regions where the dose is high and less in regions where the dose is low. Alternatively, or in addition, the sWEPL/WEPL ratio may be weighted with track end distribution, to emphasize the effects of robustness near the Bragg peaks, which are located at the track ends. The weighting may be achieved when all distributions of dose, track ends and sWEPL/WEPL are available, by calculating a measure of the amount of sWEPL/WEPL per beam or in a specific plane. The weighting is preferably done within a certain volume, for example, an outer shell surrounding the target volume, or using the track end weights as a definition of the volume.

Yet another option is to score spread of initial energy for protons stopping in a particular voxel. This is advantageous because the Bragg peaks and energy layers are a priori selected to stop at certain depths.

Using the quality value as a measure of the quality of the plan usually involves displaying the result graphically. Preferably, this involves dividing the possible value range of the quality value into intervals, each interval being assigned a colour. Each voxel could then be represented in an image by the colour corresponding to the value calculated for that particular voxel. Preferably the colour corresponding to the quality value for each voxel will be superimposed on that voxel in an image of the treatment volume. This will provide a simple graphic representation of the heterogeneity of the volume affecting the ions that stop in this voxel. Alternatively, each interval is assigned an elevation, allowing the quality values to be displayed in a 3D image.

In a simpler case, the value representing the relationship between the WEPL and the sWEPL could be compared to a threshold value to assess the plan's quality. Any voxel having a value exceeding the threshold value could be shown in a contrast colour to the rest of the image, for example red.

Depending on the nature of the radiation transport method one can chose to consider sWEPL and WEPL for only primary ions or all ions i.e. also secondary ions. In the case of a Monte Carlo based method this separation is straightforward.

The invention also relates to a computer program product comprising computer readable code means which, when executed in a computer, will cause the computer to perform the method according to the above.

The invention also relates to a non-transitory computer readable medium encoded with computer executable instructions which, when run in a first computer device will cause the device to perform the method according to the above.

The invention also relates to a computer system comprising a processor (33), a data memory (34) and a program memory (35), wherein the program memory comprises such a computer program product or non-transitory computer readable medium.

### Brief Description of the Drawings

The invention will be described in more detail in the following, by way of example and with reference to the appended drawings, in which
Figure 1 shows schematically a treatment volume comprising heterogeneities and being subjected to ion therapy.
Figure 2 illustrates the spread of water equivalent path lengths for the ions that stop in a portion of the volume
Figure 3 is a flow chart illustrating the underlying principle of the invention.
Figure 4 shows schematically a resulting image from the method.
Figure 5 is a schematic overview of a computer system that may be used to implement the invention.

### Brief Description of the Drawings

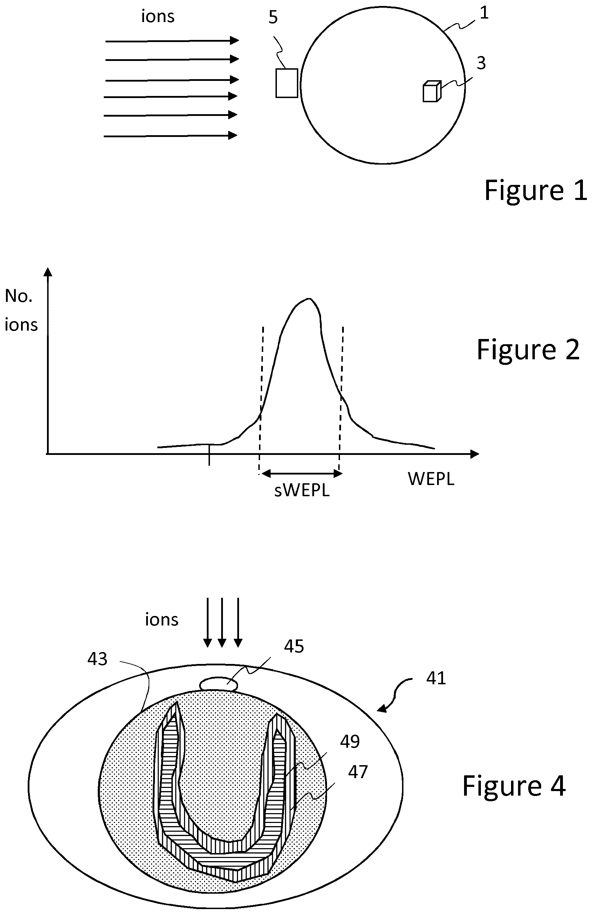

Figure 1 illustrates the principle underlying the invention by means of a simplified treatment volume 1 being subjected to incoming ions, indicated by a number of horizontal arrows. For the sake of illustration, one voxel 3 is shown within the treatment volume 1 as the voxel 3 to be considered in this discussion. As will be understood, the inventive method is carried out on a number of voxels within the treatment volume in the same way. The actual order of considering the voxels will depend on the dose calculation engine used. It should be understood throughout this document that instead of voxels in the strict sense, the method could be applied to other types of portions of the treatment volume. Normally, however, voxels are used as a suitable division of the treatment volume for practical purposes.

The treatment volume also comprises one inhomogeneous portion 5, typically a piece of bone. This inhomogeneous portion has a different density and composition from the surrounding tissue, which will cause protons passing through or near the inhomogeneous portion 5 to scatter and lose energy differently. It follows that ions reaching the voxel 5 will do so through a number of different paths through the volume 1.

For the purpose of the invention, all ions that stop within the voxel in question are considered. Their water equivalent path lengths WEPL are determined and the spread in water equivalent path length sWEPL of these ions is determined. Ions that have passed only through soft tissue having a low density will have a shorter WEPL than ions that have also passed through bone, or any other matter that has a higher density. The spread sWEPL, or the ratio sWEPL/WEPL is determined and used as a quality measure for the treatment plan. Exactly how the values for WEPL and sWEPL are determined may vary depending on a number of factors, such as the type of dose calculation engine. This is well within the capabilities of the person skilled in the art.

Figure 2 illustrates the spread of WEPL in a coordinate system showing the number of ions that stop in a particular portion of the volume as a function of the WEPL. As explained above, the portion of the volume considered is normally a voxel, but it may also be defined in a different way, such as a group of voxels. As can be seen, there is a peak around a certain WEPL value, indicating that the WEPL for most of the ions will be close to this value. The width of the peak is an indication of the sWEPL, that is, the variation in WEPL between the ions. This, in turn, is a measure of the heterogeneity as discussed above.

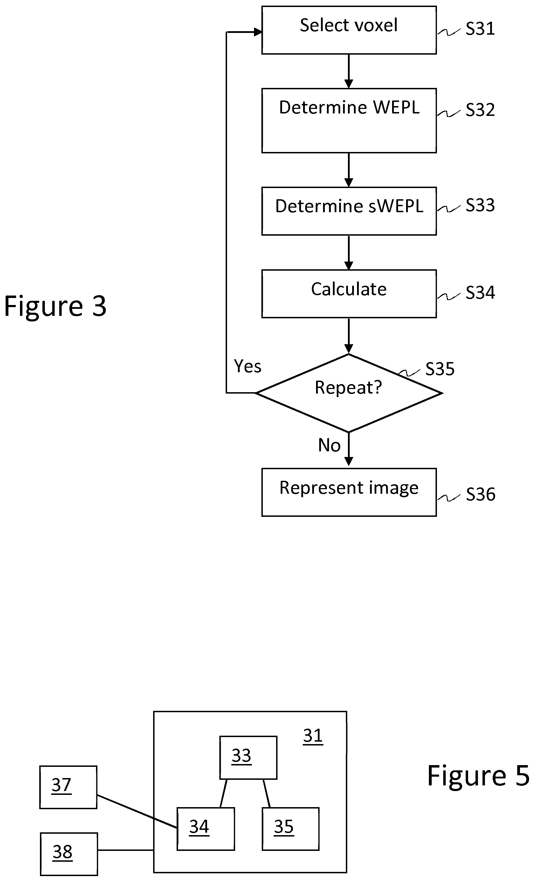

Figure 3 is a flow chart of a method according to the invention. The method is carried out separately for each portion of the treatment volume, typically for each voxel of the treatment volume.

In step S31 a voxel, or other suitable portion of the treatment volume is selected. In step S32 the water equivalent path lengths (WEPL) of all ions stopping in this treatment volume are determined. In step S33 the spread (sWEPL) between the WEPL path lengths determined in step S32 is determined. In step S34 a quantity value of a quantity indicative of the quality of the plan is calculated. This quantity value is calculated for each voxel based on sWEPL and possibly WEPL as discussed above and will be used as a quality indicator value. In step S35 it is decided if steps S1 - S4 should be repeated for another voxel. When quantity values have been calculated for all voxels, in step S36 the quantities are displayed to the user in a suitable way. Preferably, the quantities are displayed superimposed on an image representing the treatment volume. For example each possible quantity value is assigned a colour or other visual coding. The colour corresponding to the quantity value for each voxel may be superimposed on the voxel and displayed graphically, so that the assigned colour is shown for the respective voxel.

An alternative visualization method includes displaying the image as a 3d-image. In this case, instead of a colour, an elevation level is assigned to each energy level, and the elevation level corresponding to each region will be displayed superimposed on the image.

How to determine the WEPL of each ion in step S32 is known in the art and treatment planning systems commonly include modules to do this. The spread sWEPL may be determined in different ways, for example as the standard deviations of the WEPL values. Alternatively, the difference between the highest and the lowest percentiles of the WEPL values may be used as an indication of the spread, for example, the difference between the 10^{th} percentile and the 90^{th} percentile. It would also be possible to use the difference between the mean values of a certain percentage of the highest values and a certain percentage of the lowest values, respectively. A simple case would be to use the difference between the highest value and the lowest value.

It should be understood that the flow chart of Fig. 3 is presented only to illustrate the underlying idea of the invention. As the skilled person will realize, the actual order in which data items are collected and processed is not essential to the invention, and will vary with the delivery method and the dose calculation engine used in each case. Typically, when a Monte Carlo dose calculation engine is used, the calculations for all voxels will be performed in parallel and will be presented simultaneously.

Fig. 4 shows schematically a resulting image from the method. A schematic patient image 41 is shown, where the treatment volume 43 to be considered is shown as a dotted area. The ions are entering from above in the image, as shown by the vertical arrows. There is a piece of bone 45 positioned so that it will affect the path of some of the incoming ions. In creating the image, it is assumed the sWEPL has been determined for all portions of the treatment volume and a value representing the sWEPL or a relationship between the WEPL and the sWEPL has been calculated to produce a path length value for each portion.

The possible value range of the path length value has been divided into intervals, each interval being assigned a pattern. In this example, a pattern of horizontal lines indicate a very high sWEPL, or sWEPL/WEPL ratio, vertical lines indicate an elevated sWEPL or sWEPL/WEPL ratio and dots indicate an sWEPL or sWEPL/WEPL ratio within an acceptable range. As can be seen, there is an outer horseshoe-shaped area 47 behind the piece of bone 45 in which a pattern of vertical lines indicates an elevated sWEPL or ratio and an inner horseshoe-shaped area 49 having a pattern of horizontal lines indicating a very high sWEPL or ratio indicating that the ions stopping in these areas are affected by the piece of bone 45.

In a computer implemented system, the patterns in Figure 4 would normally be replaced by colours, for example using red for the very high values to indicate problem or risk, blue for areas having no risk, and green and yellow for intermediate values. Although Figure 4 shows three different levels, any suitable number of levels could be used. Also, instead of using a number of different values, the quality value for each voxel could be compared to a threshold value and all values exceeding the threshold value could be coded with the symbol or colour indicating a high quality value.

Figure 5 is a schematic representation of a computer system in which the inventive method may be performed. A computer 31 comprises a processor 33, a data memory 34 and a program memory 35. Preferably, one or more user input means 37, 38 are also present, in the form of a keyboard, a mouse, a joystick, voice recognition means or any other available user input means. The user input means may also be arranged to receive data from an external memory unit.

A treatment plan to be evaluated is found in the data memory 34. The treatment plan may be generated in the computer 31, or received from another storage means in any way known in the art.

The data memory 34 also holds sWEPL and WEPL values for each voxel. As will be understood, the data memory 34 is only shown schematically. There may be several data memory units, each holding one or more different types of data, for example, one data memory for the treatment plan, one for the CT scans, etc. The program memory 35 holds a computer program arranged to control the processor to perform the plan evaluation according to the invention.

## Claims

1. A computer-based method for determining the quality of an ion based treatment plan **characterized by** calculating, in a processor (33), for each of at least a first (3) and a second portion of a treatment volume (1; 43), a quality value indicating the spread in a path length value indicative of effective path lengths of ions that stop in that portion and to use this quality value as a measure of the quality of the plan.

2. A method according to claim 1, wherein the quality value is based on a relationship between the spread in the path length value and the path length values.

3. A method according to claim 1 or 2, wherein the path length value is based on water equivalent path lengths, WEPL, of ions that stop in the portion and the quality value is based on the spread of water equivalent path lengths, sWEPL.

4. A method according to any one of the preceding claims, wherein the path length value is based on the initial energy of each ion that stops in the portion, and the quality value is based on the spread in initial energy.

5. A method according to any one of the claims 2 - 4, wherein the quality value based on the relationship between the spread in the path length value and the path length values is obtained by calculating a weighted relationship such as dose weighted, or weighted with track end distribution.

6. A method according to any one of the preceding claims, wherein the spread in the path length value is calculated as a standard deviation of accumulated path length values.

7. A method according to any one of the claims 1 - 5, wherein the spread in the path length value is calculated as a difference in path length values between upper and lower percentiles of the path length values.

8. A method according to any one of the preceding claims, wherein the step of using the quality value as a quality measure includes:
- displaying graphically for each of the at least first and second portion of the treatment volume, information representing the calculated quality value.

9. A method according to claim 8, wherein the step of displaying information representing the quality values includes assigning a colour to each value and superimposing the colours on an image of the treatment volume.

10. A computer program product comprising computer readable code means which, when executed in a computer, will cause the computer to perform the method according to any one of the preceding claims.

11. A non-transitory computer readable medium (35) encoded with computer executable instructions which, when run in a first computer device will cause the device to perform the method according to any one of the claims 1 - 10.

12. A computer system comprising a processor (33), a data memory (34) and a program memory (35), wherein the program memory comprises a computer program product according to claim 10 or a non-transitory computer readable medium according to claim 11.

## Patentansprüche

1. Computergestütztes Verfahren zum Bestimmen der Qualität eines ionenbasierten Behandlungsplans, **gekennzeichnet durch** Berechnen, in einem Prozessor (33), für jeden von mindestens einem ersten (3) und einem zweiten Abschnitt eines Behandlungsvolumens (1; 43), eines Qualitätswerts, der die Streuung in einem Weglängenwert anzeigt, der effektive Weglängen von Ionen angibt, die in diesem Abschnitt gestoppt werden, und das Verwenden dieses Qualitätswerts als Qualitätsmaß des Plans.

2. Verfahren gemäß Anspruch 1, wobei der Qualitätswert auf einer Beziehung zwischen der Streuung in dem Weglängenwert und den Weglängenwerten basiert.

3. Verfahren gemäß Anspruch 1 oder 2, wobei der Weglängenwert auf wasseräquivalenten Weglängen (WEPL, Water Equivalent Path Length) von Ionen basiert, die in dem Abschnitt gestoppt werden, und der Qualitätswert auf der Streuung von wasseräquivalenten Weglängen (sWEPL, spread of Water Equivalent Path Length) basiert.

4. Verfahren gemäß einem der vorstehenden Ansprüche, wobei der Weglängenwert auf der Anfangsenergie jedes Ions basiert, das in dem Abschnitt gestoppt wird, und der Qualitätswert auf der Streuung der Anfangsenergie basiert

5. Verfahren gemäß einem der Ansprüche 2 bis 4, wobei der Qualitätswert, der auf der Beziehung zwischen der Streuung in dem Weglängenwert und den Weglängenwerte basiert, durch Berechnen einer gewichteten Beziehung wie etwa einer Dosisgewichtung oder eine Gewichtung mit einer Spurendverteilung erhalten wird.

6. Verfahren gemäß einem der vorstehenden Ansprüche, wobei die Streuung in dem Weglängenwert als eine Standardabweichung von akkumulierten Weglängenwerten berechnet wird.

7. Verfahren gemäß einem der Ansprüche1 bis 5, wobei die Streuung in dem Weglängenwert als ein Unterschied in Weglängenwerten zwischen oberen und unteren Perzentilen der Weglängenwerte berechnet wird.

8. Verfahren gemäß einem der vorstehenden Ansprüche, wobei der Schritt des Verwendens des Qualitätswerts als Qualitätsmaß Folgendes umfasst:
- grafisches Darstellen von Informationen, die den berechneten Qualitätswert darstellen, für jeden der mindestens ersten und zweiten Abschnitte des Behandlungsvolumens.

9. Verfahren gemäß Anspruch 8, wobei der Schritt des Anzeigens von Informationen, die die Qualitätswerte darstellen, das Zuordnen einer Farbe zu jedem Wert und das Überlagern der Farben auf einem Bild des Behandlungsvolumens umfasst.

10. Computerprogrammprodukt umfassend computerlesbare Codemittel, die, wenn sie in einem Computer ausgeführt werden, bewirken, dass der Computer das Verfahren gemäß einem der vorstehenden Ansprüche ausführt.

11. Nicht-flüchtiges, computerlesbares Medium (35), das mit computerausführbaren Anweisungen codiert ist, die, wenn sie in einer ersten Computervorrichtung ausgeführt werden, bewirken, dass die Vorrichtung das Verfahren gemäß einem der Ansprüche 1 bis 10 ausführt.

12. Computersystem umfassend einen Prozessor (33), einen Datenspeicher (34) und einen Programmspeicher (35), wobei der Programmspeicher ein Computerprogrammprodukt gemäß Anspruch 10 oder ein nicht-flüchtiges computerlesbares Medium gemäß Anspruch 11 aufweist.

## Revendications

1. Procédé informatique de détermination de la qualité d'un plan de traitement ionique **caractérisé par** le calcul, dans un processeur (33), pour chaque partie parmi au moins une première (3) et une seconde partie d'un volume de traitement (1 ; 43), d'une valeur qualitative indiquant l'écart d'une valeur de longueur de parcours indicative des longueurs de parcours effectives des ions qui s'arrêtent dans cette partie, et l'utilisation de cette valeur qualitative comme mesure de la qualité du plan.

2. Procédé selon la revendication 1, dans lequel la valeur qualitative est basée sur une relation entre l'écart de la valeur de longueur de parcours et les valeurs de longueur de parcours.

3. Procédé selon la revendication 1 ou la revendication 2, dans lequel la valeur de longueur de parcours est basée sur les longueurs de parcours équivalentes dans l'eau, WEPL (water equivalent path length), des ions qui s'arrêtent dans la partie, et la valeur qualitative est basée sur l'écart des longueurs de parcours équivalentes dans l'eau, sWEPL (spread of water equivalent path lengths).

4. Procédé selon l'une quelconque des revendications précédentes, dans lequel la valeur de longueur de parcours est basée sur l'énergie initiale de chaque ion qui s'arrête dans la partie, et la valeur qualitative est basée sur l'écart d'énergie initiale.

5. Procédé selon l'une quelconque des revendications 2 à 4, dans lequel la valeur qualitative basée sur la relation entre l'écart de valeur de longueur de parcours et les valeurs de longueur de parcours est obtenue en calculant une relation pondérée, par exemple pondérée par la dose ou pondérée par la distribution de fin de parcours.

6. Procédé selon l'une quelconque des revendications précédentes, dans lequel l'écart de valeur de longueur de parcours est calculé en tant qu'écart type des valeurs de longueur de parcours cumulées.

7. Procédé selon l'une quelconque des revendications 1 à 5, dans lequel l'écart de valeur de longueur de parcours est calculé en tant que différence des valeurs de longueur de parcours entre les percentiles supérieurs et inférieurs des valeurs de longueur de parcours.

8. Procédé selon l'une quelconque des revendications précédentes, dans lequel l'étape d'utilisation de la valeur qualitative en tant que mesure de qualité comprend :
- l'affichage graphique, pour chaque partie parmi les au moins première et seconde parties du volume de traitement, des informations représentant la valeur qualitative calculée.

9. Procédé selon la revendication 8, dans lequel l'étape d'affichage des informations représentant les valeurs qualitatives comprend l'attribution d'une couleur à chaque valeur et la superposition des couleurs sur une image du volume de traitement.

10. Produit de programme informatique comprenant un moyen de code lisible par ordinateur qui, lorsqu'il est exécuté dans un ordinateur, amène l'ordinateur à mettre en oeuvre le procédé selon l'une quelconque des revendications précédentes.

11. Support non transitoire lisible par ordinateur (35) codé avec des instructions exécutables par un ordinateur qui, lorsqu'il est lu dans un premier dispositif informatique, amène le dispositif informatique à mettre en oeuvre le procédé selon l'une quelconque des revendications 1 à 10.

12. Système informatique comprenant un processeur (33), une mémoire de données (34) et une mémoire de programme (35), dans lequel la mémoire de programme comprend un produit de programme informatique selon la revendication 10 ou un support non transitoire lisible par un ordinateur selon la revendication 11.
